# EUROPEAN PATENT SPECIFICATION

(11) **EP 1 861 101 B1**
(45) Date of publication and mention of the grant of the patent: **24.10.2012**
(21) Application number: 06725231.2
(22) Date of filing: 22.03.2006
(51) Int. Cl.: A61K 31/506, A61P 1/00

(54) **USE OF AMIDES OF SULFADIMIDINE FOR THE TREATMENT OF INTESTINAL DISEASES IN VETERINARY SCIENCE**
VERWENDUNG VON AMIDEN VON SULFADIMIDIN ZUR BEHANDLUNG VON DARMERKRANKUNGEN IN DER VETERINÄRMEDIZIN
UTILISATION D'AMIDE DE SULFADIMINIDE POUR TRAITER DES MALADIES INTESTINALES DANS LES DOMAINES MÉDICAL ET VÉTÉRINAIRE

(30) Priority: 23.03.2005 EP 05102365; 10.02.2006 EP 06101509
(43) Date of publication of application: 05.12.2007
(73) Proprietor: Klat-Pharma Animal Health GmbH, 27798 Wuesting (Hude) (DE)
(72) Inventor: MASSAGUE, Antonio J., E-08017 Barcelona (ES); MASSAGUE, Joan, E-08013 Barcelona (ES)
(74) Representative: von Kreisler Selting Werner
(86) International application number: PCT/EP2006/060948
(87) International publication number: WO 2006/100263

(56) References cited:
- WO-A-03/008387
- GB-A- 1 217 936
- US-A- 3 395 141

## Description

The invention relates to a treatment of diseases of the digestive tract of animals, including humans, using a compound which is a dicarboxylic acid amide of sulfadimidine, and medicaments and food and beverage preparations comprising the compound.

In animal farming various diseases are known caused by bacteria, parasites or mixed infections. The diseases occur more frequently and severe under conditions, where the animals are stressed, amongst which are high density of animals, high reproduction rates, deficiencies in feeding, high humidity or temperature. Since in intensive farming the diseases easily spread amongst the animals, they cause high economic losses for the farmers.

Various antibiotics and chemotherapeutics are known and widely used, depending on the type and age of animals, the breeding conditions and the national provisions for farming. The use of such substances causes many problems, amongst which are side-effects for the animals and consumers, accumulation of the substances in meat, milk and eggs, the development of resistances of microorganisms and the high costs of many therapeutics. The problem underlying the invention is thus to provide an effective treatment of various diseases in animals caused by bacteria or parasites. The treatment should preferably be easily available and applicable, should not cause undesirable side-effects, and should affect the meat or the eggs of the animals little as possible.

US-A-3,395,141 discloses the preparation of sulfamethazine and acetyl sulfametnezine. The purity and yield of sulfamethazine or acetyl sulfamethazine from the reaction of sulfaguanidine or acetyl sulfaguanidine and 2,4-pentanedione (acetylacetone) is improved by reacting in an aqueous acidic buffered solution (pH 3.0 to 6.0) with the beta-diketone in excess, and at approximately the boiling point. A longer chain diketone may be used for corresponding other 2-p-amino-benzenesulfonamido-4,6-dialkylpyrimidines. Sulfamethazine and acetylsulfamethazine are pharmaceutical specialities. Sulfamethazine is frequently prescribed for the treatment of infections, and acetylsulfamethazine is readily hydrolyzed to sulfamethazine.

WO-A1-03/008387 relates to compounds of formula (I) or a pharmaceutically acceptable salt thereof wherein R₁, R₂, R₃, R₄ and R₅ are independently of each other H, a straight or branched alkyl or alkoxy group with 1 to 4 carbon atoms, or a halogen; X is H, a straight or branched alkyl chain with 1 to 4 carbon atoms, phenyl, -OH or =O; Z in H, acetyl, -CH₂-Ph-O-CF₃ or CH₂-Ph-CF₃, Y is a ring structure optionally linked to formula (I) with an alkyl chain having one or two carbon atoms. This invention further relates to the use of said compounds for the manufacture of a pharmaceutical preparation useful for the treatment or prevention of a disease mediated by the alpha-2B-adrenoceptor in a mammal.

The specific state of the art, problems and requirements, exemplified for the farming of poultry, rabbits, ovine livestock, swine and bovine livestock, are discussed in detail in following subsections of the description.

Surprisingly, the problem is solved by the compounds and medical, food and liquid preparations, uses and methods according to claims 1 to 22.

Subject of the invention are compounds and medical preparations for the treatment of diseases of the digestive tract of animals, including humans, comprising a dicarboxylic acid amide of sulfadimidine, wherein the amide is in particular essentially non-absorbable in the intestine. As these compounds are not absorbable, it is possible to get high concentrations of them in the intestinal tract, without a simultaneously high blood level concentration and consequently without toxic effects. The medicaments, food and beverage compositions of the invention are preferably for veterinary use.

Subject of the invention is further a food preparation for animals comprising a dicarboxylic acid amide of sulfadimidine wherein the amide is essentially non-absorbable in the intestine, wherein the amide is admixed with the food. Preferred are food preparations, wherein the amide is comprised at amounts of 0.1 to 2 g/kg, more preferably 0.5-1 g/kg of feed.

Subject of the invention is further a beverage preparation for animals comprising a dicarboxylic acid amide of sulfadimidine wherein the amide is essentially non-absorbable in the intestine. Preferably, the beverage is a milk substitute.

Sulfadimidine is also known under various other names, amongst them sulfamethazine and 2-(4-aminobenzenesulfonamido)-4,6-dimethylpyrimidine. As used herein, an "amide of sulfadimidine" is a compound, wherein sulfadimidine is linked to an acid via an amide bond. Specifically, the acid is linked to the sole amino group of the sulfadimidine via an amide bond. The compounds of the invention thus consist of a sulfadimidine radical (moiety) and an carboxyacyl radical (moiety) linked by the amide bond. Both moieties might be derivatised. As used throughout the application, the carboxyacyl radical is designated by replacing the terminal "-ic" in the name of the acid by the ending "-yl" as, for ex-ample, the succinyl, oxalyl, malonyl, phthalyl, maleyl, adipyl, glutaryl, aspartyl, tartaryl, fumaryl, mesaconyl, and citryl carboxyacyl radicals and the like, derived respectively from succinic, oxalic, malonic, phthalic, maleic, adipic, glutaric, aspartic, tartaric, fumaric, mesaconic, and citric acids and the like.

Preferably, the compound is comprised at concentrations of 0.05 to 1 g/l, more preferably 0.25 to 0.55 g/l.

Subject of the invention is also the use of a dicarboxylic acid amide of sulfadimidine for the preparation of a medicament against diseases of the digestive tract in animals, wherein the amide is essentially non-absorbable in the intestine.

According to the invention, the compound is the amide of a dicarboxylic acid. Especially preferred is sulfadimidine maleic acid amide (SMAA).

In preferred embodiments of the invention, the compounds are essentially soluble or essentially insoluble in water. If the amide is the amide of maleic acid (SMAA), the amide comprises one carboxylic group. The free acid of SMAA is essentially insoluble in water, whereas the sodium salt is essentially soluble. The free acids of the compound of the invention are essentially water insoluble and electrically neutral compounds, which become soluble in water when the free carboxyl group or other groups in the compound are converted to the carboxylate form by addition of an equivalent amount of an appropriate base, for instance an alkali or ammonium hydroxide or amine or alkanolamine.

Especially the sulfadimidines, in which the carboxyacyl radical is aliphatic and contains four carbon atoms in the aliphatic chain, are effective against intestinal diseases.

In the compounds of the invention, the sulfadimidine may be replaced by a derivative of sulfadimidine. In the derivatives, the sulfadimidine might be altered at the carboxyacyl group, the sulphanilyl group or the aminopyrimidine. In the derivatives, the one or both of the aromatic rings of the sulfadimidine might be modified, preferably with a methyl, ethyl, propyl, amino, fluoro, chloro or bromo group. However, the derivative of sulfadimidine is selected such that the compound is essentially non-absorbable in the intestine. Besides, the compound should preferably have a therapeutic activity not lower than that of a comparable compound with sulfadimidine, and at least 10%, more preferably 20 or 50 % of this activity.

The carboxyacyl moiety is preferably derived from a polycarboxylic acids, more preferably a dicarboxylic acid. In a preferred embodiment of the invention, the compound comprises an aliphatic side chain as the carboxyacyl moiety. Preferably, the aliphatic side chain consists of 3 to 8, more preferably 3, 4, 5 or 6 carbon atoms, including the carboxylic group. The side chain may be saturated or unsaturated.

Among the particularly effective compounds are those in which the aliphatic chain is saturated, such as those in which the carboxyacyl radical is derived from succinic, malic (the three isomeric forms), aspartic, citromaleic, pyrotartaric, ethylsuccinic, tartaric and ketosuccinic acids and their substituted derivatives, and those in which the aliphatic chain is unsaturated, such as those in which the carboxyacyl radical is obtained from maleic, citraconic, fumaric and mesaconic acids and their substituted derivatives.

Preferably, the absorption of the amide in the intestine is less than 0.01% or 0.005%, more preferably less than 0.002% or 0.001%. "Absorption" in this context means, that a compound is taken up by the intestinal wall and passes the intestinal wall.

The amides of sulfadimidine are obtainable by amidation of sulfadimidine according to methods of organic synthesis known in the art.

In specific embodiments of the invention, the diseases treated are caused by or associated with the infection of the digestive tract by parasites and/or bacteria. The diseases may be intestinal coccidiosis, digestive syndrome, enterotoxemia (overeating disease), dysentery, enteric colibacillosis (watery diarrhea), an infection by *Salmonella, E. coli, Streptococcus, Staphylococcus* and/or *Proteus* and/or diseases caused by infection with *Coccidia, Eimeria* and/or *Clostidium perfingens*.

Amongst the animals treated or fed according to the invention are pigs, rabbits, poultry, especially chicken, turkey and/or ruminants, especially cattle, sheep.

The compounds of the invention, used as intestinal antiseptics, are preferably administered orally, either in the form of tablets, capsules or powders of the solid material, food or beverage additives, or as solutions of any desired concentration, exceeding, if desired, 50% (w/v).

In an embodiment of the invention the amide is applied in combination with at least one other antibiotic and/or chemotherapeutic agent. The other antibiotic or chemotherapeutic agent is preferably selected from the group consisting of chlortetracycline, oxytetracycline, colistin, tetracycline, tylosin, gentamicin or neomycin.

In one embodiment, the preparation of the medicament of the invention comprises dissolution of the amide of sulfadimidine in water, a beverage for animals or a milk substitute. In another embodiment, the preparation of the medicament comprises addition of the amide of sulfadimidine to food.

The medicament of the invention is essentially non-absorbable by the intestine. Therefore it has maximum activity throughout the digestive tract. No renal toxicity is observed, even in species sensitive to sulfonamides. Besides, the medicament does not affect reproductive parameters (fertility, fecundity, etc.). No accumulation of residues in products for human consumption (eggs, milk, and meat) occurs. In milk-producing animals the absence of residues in the milk facilitates the preparation of cheese because it does not interfere with fermentation. The medicament does not require a withdrawal period. Lower doses can be used without diminishing the efficacy of treatment.

SMAA as opposed to other antibiotics and chemotherapeutic agents used in the digestive tract (neomycin, colistin, furans, etc.) has a broad spectrum of activity. It is active against Gram-negative and Gram-positive microorganisms that colonize the digestive tract, amongst which are *Clostridium, Salmonella, Escherichia coli, Streptococcus, Staphylococcus, Proteus* It is also effective against *Eimeria* (coccidiosis).

It may be applied as a solid or in solution. It is bacteriostatic and favors the development of immunity by the animal. The possibility of resistance is lower than for known antibiotics. It is an economical, advantageous alternative to furans, dimetridazole, olaquindox, and carbadox in countries where the use of these therapeutic molecules has been prohibited. It functions synergistic with other antibiotics such as neomycin or colistin and can be applied in combination with other chemotherapeutical agents.

An incompatibility with other medicaments is unknown. Since the product is not absorbed, it does not leave residues in meat, eggs or milk, so no withdrawal period is necessary. If associated to antibiotics, the withdrawal period of the antibiotic must be considered. In soluble form, a dosage of 500 to 1100 g/2000 liters of drinking water is preferred. The treatment preferably is applied for 4 to 6 days.

Added to milk substitute, from 5 to 8 g/kg of milk substitute, to provide a maximum daily supply of 400 g per calf each day, or 4 liters at a concentration of 100 g/litre of water.

### Poultry farming

In poultry farming, the food preparation and medicament of the invention is especially useful in the treatment of Coccidiosis, enteric Colibacillosis, Salmonellosis and diseases associated with other bacteria of enteric location (*Clostridia* etc.)

### Etiology of Coccidiosis:

Coccidiosis is a parasitic disease produced by different species of protozoa of the *Eimeria* genus, which multiply in the intestinal tract causing intestinal damage and deterioration of nutritional conversion, slowed growth, increased mortality, bloody diarrhea and dehydration. In addition, it enhances the sensitivity of the animal to other pathogens, such as *Clostridium perfringens* or *Salmonella typhimurium.*

The pathology of coccidiosis must be understood from two vantage points: When the disease appears abruptly, it causes many casualties (clinical signs). When the infection is not sufficiently intense to cause symptoms, but significantly affects productivity, etc. (subclinical signs).

Like other parasitic infections, coccidiosis is a disease that has a greater incidence in young animals because their immune systems develop after exposure to the parasite, protecting them against later infections. Nonetheless, there is no cross-immunity between species of *Eimeria,* which is why outbreaks that may appear later are due to other species.

The high density of animals in farms, humidity and temperature, and the elevated reproductive potential of *Coccidia* increase the incidence of this parasitic infestation in all types of farming operations. This makes it necessary to use coccidiostatics continuously, and to alternate them during a single productive cycle to prevent infection or to reduce its incidence on the growth or production of poultry.

Various drugs are known for the treatment of coccidiosis. A series of factors must be considered when choosing the drug to be used, whether to treat or prevent an outbreak of coccidiosis. Sulfonamides compete for PABA uptake in folic acid metabolism. Amprolium competes for thiamine absorption with the parasite. Quinolines and clopidol inhibit the energy metabolism of the *Coccidia* cytochrome system. Ionophores change the osmotic balance of protozoan cells by modifying the permeability of the cell membrane to alkaline metal cations.

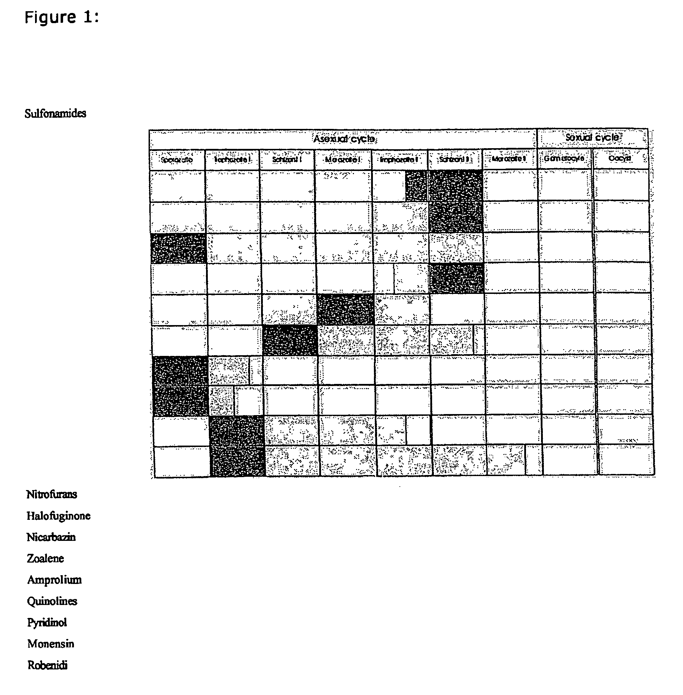

Each species of *Eimeria* is characterized, among other factors, by its colonization of a specific zone of the intestinal tract. The drug selected should be able to reach the zone while conserving maximum activity. As can be appreciated in the diagram in figure 1, each drug is active against specific stages of parasite development. Since the development of immunity is the best weapon available for combating disease, it is important to use products that favour the generation of immunity. Immunity is created during Schizont I and Merozoite I and II.

### Non-absorbable sulfonamides in the treatment of coccidiosis:

To improve the digestive efficacy of sulfonamides, the use of non-absorbable derivatives according to the invention has the following advantages.

The drugs are active throughout the entire intestinal tract. This improves their efficacy against *Eimeria* located in the second half of the intestine and cecum, which usually are more virulent. The medicament of the invention is not toxic. As sulfonamides that do not pass into the bloodstream, the possibility of intoxication with renal crystalluria is avoided. Therefore, they are extremely safe products. As bacteriostatic agents they facilitate the development of immunity: This prevents later reinfection of broilers or achieves the immunization of chicks destined for reproduction, when these non-absorbable derivatives are administered during the breeding and rebreeding phases of young chicks. Unlike base products, non-absorbable derivatives do not leave residues in meat, because they do not pass into the bloodstream.

Their use in reproductive hens affects neither egg-laying nor egg incubability because these products act exclusively on the intestine.

### Field tests:

SMAA was used for the treatment of coccidiosis in broilers through feed, at doses ranging from 200 to 400 g/metric ton of feed.

The best results in terms of mortality, weight gain, and conversion index have been obtained when SMAA is administered in the first three weeks of life of the chickens, and then replaced by another coccidiostatic. To this effect, our experience confirms the observations that an even improved productivity is obtained when two different coccidiostatics are alternated in the growth period.

### Rabbit farming

### Intestinal Coccidiosis:

The multiplication of *Coccidia,* common parasites of the rabbit intestine, causes this serious disease, which generates important economic losses. According to several authors, there are more than ten different *Coccidia,* each of which causes a particular infection of variable severity: From very pathogenic species, such as *E. flavescens, E. intestinalis,* and *E. piriformis,* which can produce high mortality rates in the absence of the intervention of other synergistic factors, to moderate or mildly pathogenic organisms, such as *E. media, E. magna,* and *E. perforans,* which owe their dangerousness to the fact that they reduce the defenses of the animal, thus favoring the entrance of other microorganisms that are more pathogenic than *Eimeria.*

The usual process of development of coccidiosis occurs is the following: A nonspecific aggression occurs, which might be physical (transportation, noise, environmental and temperature changes, stress, etc.), chemical (air excessively laden with ammonia, heavy gases, medications, etc.) or biological (weaning, dense microbial environment, change of diet, respiratory disease, etc.). An exhaustion of the organism's capacity to react is observed, followed by development of *Coccidia* that induce coccidiosis. A Multiplication of large amounts of *Coccidia* occurs that become agents of disease *per se* as a result of persistent infection. Eventually, colibacillary development appears.

The biological cycle of the parasite has two different phases: an internal phase (schizogony, gametogony) characterized by parasitic multiplication with expulsion of oocysts, and an external phase (sporogony) during which oocysts mature and become a source of dissemination. This suggests that hygienic measures (keeping surroundings dry, disinfection) are extremely important in the fight against this disease.

When considering anticooccidia therapy, the following points must be weighed: The success of treatment will depend to a great extent on the speed of action because the severity of the disease increases with the number of infective elements. Anticoccidiotics generally act on a phase of the developmental cycle of *Eimeria,* so prolonged treatments are necessary to achieve real efficacy. The administration of sulfonamides at therapeutic doses in the first two weeks after weaning prevents the development of hepatic coccidiosis. Traditional absorbable sulfonamides, when administered for a prolonged period, can give rise to problems of nephrotoxicity. This drawback is avoided by the method of the invention using non-absorbable sulfonamides, SMAA, that also improve anticoccidiotic activity by acting exclusively on the intestinal tract.

Table 1 shows the results obtained with anticoccidiotic treatments with base sulfonamides and their non-absorbable derivatives:

**Table 1**

| Medication | Dosage | Observations |
|---|---|---|
| Sulfadimethoxine | 75 to 200 mg/kg live weight for 3 to 7 days. | Very interesting in mixed infections, but requires a period of withdrawal before slaughter. |
| Sulfadimethoxine maleic acid amide | 0.2 to 0.5 g/kg feed | Very interesting in mixed infections. Treatments can continue for 21 days without risk of toxicity or residues in carcasses. |
| Sulfadimidine | 0.8 to 1 g/l of water for 3 to 5 days. | This treatment curtails mortality. It is recommended to continue this treatment not more than 5 days. |
| Sulfadimidine maleic acid amide (SMAA) | 0.3 to 1 g/kg of feed | It is most effective in mixed infections with *E*. *coli.* Treatment can be extended up to 3 weeks, if given in low doses, without risk of toxicity or residues in carcasses. |

### Digestive Syndrome:

*E. coli* is an organism that is usually present in the digestive tract of rabbits, which usually at most causes banal diarrhea due to excessive proliferation that ceases when the cause disappears. However, due to pathogenic strains the disease Colibacillosis occurs, which is very important in rabbit farms. The disorder is frequent in the neonatal period during mixed feeding (milk + solid food), in the phase subsequent to weaning, and during growth. Pathologic manifestations of *E. coli* proliferation are triggered or exacerbated by factors such as local reduction of acidity, concomitant coccidiosis, excess dietary protein, and others. Colibacillosis is transmitted by contact and it appears in the neonatal phase of conies due to contamination by the doe. For this reason, washouts should be performed before delivery.

The toxi-infectious disease enterotoxemia is produced by anaerobic organisms pertaining to the genus *Clostridium* that inhabit the intestine of the rabbit. They usually are present acutely and affect mainly adult animals, although they can also occur in young animals, particularly during weaning. The incidence is highest between the months of January and June. Symptoms appear when *Clostridium perfringens,* habitually found in the microflora of the rabbit, multiplies without control due to a series of predisposing causes, such as deficiencies in rationing and feeding (feed overly rich in carbohydrates or proteins and poor in fiber), certain renal lesions caused by chronic disease or after prolonged treatment with traditional absorbable sulfonamides and/or the administration of antibiotics, like penicillin, clindamycin, and lincomycin, which cause cecal dysbacteriosis.

Given the complex relations existing between colibacillosis and enterotoxemia, they are currently included under the designation "digestive syndrome" for practical purposes. Treatment with an association of systemic drugs with drugs acting exclusively on the intestinal tract is indicated.

In the following table 2 the use of SMAA is compared to the products used most often in rabbit farming for the prevention and treatment of digestive syndrome:

**Table 2**

| **Medication** | **Dosage** | **Observations** |
|---|---|---|
| Colistin | 50 to 80,000 IU/kg p.v. per day, for 5-7 days | Colistin reduces mortality |
| Enrofloxacin | 50 mg/1 of drinking water for 3 days. | Effective |
| SMAA | 0.30 to 0.5 g/kg feed | The most effective agent in mixed infections by *Coccidia.* Treatment can be prolonged without risk of toxicity. This treatment curtails mortality. |
| Furazolidone | 200 to 250 g/ton of feed | Effective. |
| Neomycin | 250 to 300 mg/l of drinking water | Efficacious against moderately pathogenic strains. |
| Tetracycline | 300 to 500 mg/l of drinking water | Efficacious against moderately pathogenic strains. |

In summary, the results show the importance of non-absorptive amides of sulfonamides in the treatment and prevention of infectious digestive processes in rabbits. Intestinal coccidiosis and enteric colibacillosis and enterotoxemia are treated effectively in feed with SMAA and in water with soluble SMAA.

### Ovine livestock

### Coccidiosis:

Coccidiosis is a protozoal infectious disease caused by *Coccidia* species of the genus *Eimeria,* which reside preferentially in the posterior segments of the small intestine. Although it is a disease that does not cause serious damage in adult animals, because they acquire immunity with age, adults are responsible, as *Coccidia* carriers, for infecting lambs, in which the development of coccidiosis can cause many growth-related problems that will directly affect farm profits. The animals at greatest risk are young lambs, 6 to 12 weeks-old, that live with breeding ewes, generally under conditions of confinement in a stable with high humidity and density of animals, etc.. Coccidiosis outbreaks are observed mainly towards the end of winter and beginning of spring.

Although there are molecules that have coccidiostatic activity, none of them is authorized for use in lambs, which makes disease prevention difficult and requires the use of medicated feed. Of the antibiotics and chemotherapeutic agents habitually used in ovines, only sulfonamides have activity against *Coccidia,* making SMAA use according to the invention indispensable. In addition to its efficacy in the treatment of coccidiosis, it does not require a withdrawal period.

In a preferred embodiment of the invention, coccidiosis is treated at two levels: As mentioned above, adult ewes are responsible for infecting lambs. Therefore, to minimize the risk of transmission, pregnant ewes should be treated with SMAA in the 15 to 20-day period before dropping and up to 5 to 10 days after dropping. To act on the most susceptible animals (young lambs) and at the times of year in which the likelihood of the problem appearing is highest, the addition of medication to the "starter" feed of lambs is preferred, which is administered for two weeks after weaning. If an outbreak of coccidiosis occurs during the growth period, treatment with SMAA is not a problem because it does not leave residues in carcasses.

### Enterotoxemia (Overeating disease):

Enterotoxemia is produced by the toxin of *Clostridium perfringens* type D, which is found in the digestive tract of ruminants. Like other regular inhabitants of the digestive tract, under normal conditions they are no risks, but the coincidence of factors favorable to overproliferation predisposes to the development of disease. Predisposing factors include situations of nutritional stress (intensive feeding, excess intake, imbalances between energy intake and protein content of feed, etc.) and environmental stress (cold, abrupt changes in temperature, etc). Since it is often difficult to avoid predisposing factors, it is necessary at certain times of the year to administer chemotherapeutics or antibiotics to control the presence of *Clostridium* in the digestive tract of lambs. Sulfonamides and tetracyclines are especially active against *Clostridium,* which is why preventive treatment with SMAA, alone or associated with a tetracycline, is advisable. In pregnant ewes the disease is controlled with the treatment for coccidiosis according to the invention. In lambs, the condition is controlled by adding medication to the starter feed because SMAA is sufficient and does not require a withdrawal period.

### Enteric colibacillosis (Watery diarrhea):

This infectious disease is produced by *Escherichia coli.* Other microorganisms can break out and complicate the condition, such as *Staphylococci, Streptococci, Proteus, Enterococci, Shigella, Clostridium,* etc. It is a disease that appears more often in winter and can be intensified by predisposing factors like suppression of calostrum intake, confinement in poorly ventilated stables, mastitis, and others and depends on the enteropathogenic nature of the various *E*. *coli* serotypes. It affects mainly nursing animals in the first 1-5 weeks of life, which is why, as noted above, preliminary treatment of pregnant ewes just before dropping is interesting. According to the invention, the addition of medication to the starter feed for lambs is also very efficacious. Due to the large variety of *E*. *coli* serotypes and their different sensitivities to antibiotics and chemotherapeutics, it is common to resort to the association of two molecules.

### Efficacy tests of SMAA (sulfadimidine amide of maleic acid):

Enterotoxemia is produced by the toxin of *Clostridium perfringens* (type D), which is habitually found in the digestive tract of ruminants. Among the factors predisposing to the disease are intensive feeding (feed rich in energy and protein), overeating, changes in feeding, cold, etc. It is difficult to avoid the action of predisposing causes. Consequently, it is advantageous to choose drugs according to the invention that do not leave residues in carcasses and have a period of withdrawal compatible with livestock management.

A trial was performed in order to study the effect of continued medication with feed containing SMAA on the incidence of *C*. *perfringens* (enterotoxemia) in the growth diet of lambs. Five thousand lambs were tested in an intensive operation. For the duration of the trial (4 weeks) there was a constant entry and exit of animals for growth and finishing. Therefore, there was always a possibility of contagion due to the arrival of animals of different origins, as well as poor adaptation of the animals that were received to the microbial environment of the operation.

On the other hand, due to market needs and a greater than usual demand for lambs, animals were fed a richer feed (1.03 UFC) to force growth. These two conditioners (constant entrance and exit of animals and high energy feeding) under normal conditions would increase the incidence of enterotoxemia.

SMAA was administered at a dosage of 500 g/ton of feed. At the end of the trial, enterotoxemia was absent in the livestock and the mortality was lower than 0.3% and due to undetermined causes. Likewise, no case of watery diarrhea caused by *E. coli, Salmonella* dysentery, or coccidiosis occurred.

The trial shows that SMAA is a therapeutic agent of choice for preventing mortality due to enterotoxemia and, in general, for controlling digestive pathology common in intensive lamb production. It improved productivity indices while offering the advantage of not leaving residue in meat and thus eliminating the need for a withdrawal period.

### Reproduction pathology: salmonella abortion

Although abortions are not common, the most frequent cause is the proliferation of salmonella, which are regular inhabitants of the digestive system. The reduction of corporal defenses due to situations of stress, other diseases, and other factors facilitates the multiplication of these bacteria and their transmission to the bloodstream of affected pregnant ewes and from ewe to fetus. Abortions generally occur late in gestation (4 to 2 last weeks), although fetuses are sometimes born prematurely alive. Nevertheless, the vitality of these lambs is so low that most die within 24 - 48 hours. Since it is difficult to find animals free of *Salmonella* because it is a habitual inhabitant of the digestive tract, the best way to prevent *Salmonella* abortions is to carry out periodic washout treatments of ewes that have a bacterial level that could lead to transmission to the bloodstream.

Salmonella is sensitive to the action of sulfonamides and tetracyclines, which is why the association of SMAA with tetracyclines would be the treatment of choice for washout:
Preferably, 0.3 kg/ton of SMAA should be administered in combination with 0.2 kg/ton of chlortetracycline. The treatment should be given during days 85 to 135 of gestation.

### Summary:

In general, ovine livestock according to the invention is preferably treated with SMAA in food. The invention is especially useful for the prevention of enteric bacterial processes, mainly due to *Clostridium, E. coli, Salmonella,* and *Eimeria* parasites. Given the absence of renal toxicity of these derivatives (when not absorbed, they do not enter the bloodstream), treatment can be prolonged for several weeks without observing adverse effects. Even at high doses, no problems of dysbacteriosis have appeared. Incompatibilities with other medicaments are unknown. It is not necessary to establish a withdrawal period before slaughter. Studies of the depletion of residue in lambs that have consumed feed treated with SMAA until the day of slaughter demonstrate that the residue level in meat is below 30 ppb (The MRL for sulfonamides is 100 ppb).

Further experiments demonstrated that SMAA can be associated to chlortetracycline and oxytetracycline with very good results. According to the invention, the association of SMAA-chlortetracycline is preferred when the pathology is located fundamentally in the digestive tract, and the association of SMAA-oxytetracycline when a preventive action at the enteric and respiratory level is sought. The most habitual doses range from 200 and 400 g/ton of feed, although it can be raised to 750 g/ton without problems.

In another preferred embodiment of the invention, soluble SMAA is applied with drinking water. This application is especially useful for the treatment of enteric bacterial processes, mainly *Clostridium, E. coli,* and *Salmonella.* The treatment is highly effective for the treatment of coccidiosis outbreaks. The treatment is usually administered for a period of 4 to 6 days, although it can be prolonged due to the absence of renal toxicity of these derivatives (since they are not absorbed, they do not enter the bloodstream), without observing adverse effects. Even at high doses, no problems of dysbacteriosis have appeared. Incompatibilities with other medicaments are unknown. A withdrawal period before slaughter is not necessary. When non-absorbable derivatives of sulfadimidine are added to drinking water, they are not often associated with other molecules. If other molecules are associated, drug incompatibilities usually do not occur because the pH of the solution of SMAA soluble is preferably not alkaline. The usual dose ranges from 550-1100 g/2000 liters of water for 4 to 6 days. The availability of a soluble form makes it possible to administer the product in a milk substitute. In this case the dosage should be established in relation to the consumption of powdered milk substitutes.

### Swine livestock

### Enteric Processes:

Problems of enteric location in suckling pigs occur mainly in the period between weaning and approximately 60 days of life. The main causes are, on the one hand, the as yet insufficient development of the immune system of the animals, which allows the development of the microbes that cause digestive syndrome in pigs and, on the other hand, the lack of adaptation of digestive enzymes to the very early change from nursing to feed.

There exists a general increase in infectious processes in the intestine during the growth period. Their etiology varies and this process can be included under the designation of the so-called intestinal syndrome. The cause of its increasing frequency seems to be related, among other possible factors, to the increasing use of certain raw materials in feed formulations, mainly mandioca, gluten feed, etc. whose microbial content is often high. The difference in price between these raw materials and barley and wheat byproducts has led to their use with the aim of obtaining a growth feed at a competitive price. The causes of digestive bacterial diseases in pigs, either in the starter phase or in the growth stage of the animal, are multiple and varied. However, the predominant causes are *Escherichia coli, Salmonella, Campylobacter, Clostridium, Treponema, Coccidia* and microbes arising as a complication (*Streptococcus, Staphylococcus,* etc.)

The sensitivity of these microbes to sulfonamides is generally good. Therefore, it is correct to consider the use of sulfonamides for the prevention or treatment of digestive processes because they are broad-spectrum molecules. Nonetheless, it is always advisable to associate them with other antibiotics or chemotherapeutic agents to improve the efficacy of treatment and prevent the proliferation of strains that have developed resistance to them.

### SMAA in the starter feed of suckling pigs:

There are a number of references in the literature that recommend the use of sulfadimidine in the pre-starter and starter feed of suckling pigs, alone or in association with other antibiotics and chemotherapeutic agents. This reflects the sensitivity of microorganisms that cause digestive syndrome to this sulfonamide. However, the digestive efficacy of sulfadimidine can be improved by using its non-absorbable derivative. In effect, SMAA makes it possible to improve the digestive efficacy of sulfadimidine by avoiding the absorption and passage into the bloodstream of part of the dose taken by the animal. This concentrates all the activity in gastrointestinal tract.

The advantages are:
- Maximal activity throughout the gastrointestinal tract.
- Possibility of reducing the dose and thus realizing a savings in medication costs.
- No risk of renal intoxication, even when used at high doses, due to the absence of intestinal absorption.
- Due to its spectrum of activity, SMAA is an efficacious alternative to furans that, until prohibited, were widely used in starter feeds.

### SMAA in growth feed:

In the present situation growth feeds are formulated from low-cost raw materials to offer livestock owners feed at competitive prices. This carries the risk of increasing the bacterial load of the feed and favoring the development of microbes that can cause digestive syndrome as has been appearing in the growth phase.

On the other hand, the livestock owner is forced to use medicated feed that can be administered until advanced phases of the growth period without the risk of exceeding the maximum residue limit for each substance. For that reason, the use of sulfadimidine is not recommended because this molecule has a withdrawal period of 28 days. According to the invention, without risk of leaving residues in carcasses the non-absorbable derivative SMAA, is advantageous.

For these reasons, the use of SMAA has the following advantages:
- Possibility to administer medicated feed until slaughter because it does not leave sulfadimidine residues in carcasses.
- Its broad spectrum of activity, which includes Gram-positive and Gram-negative bacteria, and *Eimeria* protozoa, which are responsible for infectious digestive processes during the growth phase.
- Maximal activity throughout the gastrointestinal tract.
- Dosage lower than that of sulfadimidine, resulting in a savings of medication costs.
- No risk of renal intoxication, even when used at high doses, due to the absence of intestinal absorption.
- Due to its spectrum of activity, SMAA is an effective alternative to the furans that, until their prohibition, were widely used in feed for swine.

The invention comprises the inclusion in the gestation/lactation feed of reproducers, to reduce the incidence of mastitis-metritis-agalactia complex in sows and neonatal diarrhea in sucklings.

Although it is always difficult to establish a dose for the use of a therapeutic molecule, because therapeutic efficacy does not depend only on its efficacy at a given dose, but also on the state of the animals, management, sanitary conditions of operation, feeding, etc., good results were obtained with SMAA in swine at 400-600 g of SMAA per ton of feed.

In some cases the association of SMAA with other antibiotics or chemotherapeutic agents can be interesting. In light of the performed tests preferred are the use of non-absorbable molecules like colistin or neomycin when the problem is exclusively digestive, and tetracycline or tylosin when a respiratory component is associated with the enteric process. In these cases it is extremely important to consider the withdrawal period of absorbable molecules to avoid the accumulation of residues in carcasses.

### Swine dysentery:

Swine dysentery, vibrionic dysentery, or bloody diarrhea is a disease produced by an anaerobic spirochete, *Treponema hyodisenteriae,* now better known as *Serpulina hyodisenteriae.* The disease was described in 1921, but its true etiology was not established until 1971 by Taylor and Alexander of the University of Cambridge. Its causal agent was initially considered *Vibrio coli* and later a spirochete, *Treponema hyodisenteriae.* However, Sellwood (1991) confirmed that the causal microorganism did not have the typical characteristics of *Treponemas* and in 1992 Stanton classified it as *Serpulina.* Among these secondary agents, *Bacteroides vulgatus, Fusobacterium necrophorum, Balantidium, Bordetella, Mycoplasma* and *Campylobacter coli* merit mention. *Campylobacter* is also considered a primary agent and its denomination corresponds to the old *Vibrio coli.*

The disease appears in all types of pigs, especially those weighing 15 to 70 kg. It is also common, although its course is not apparent, in reproductive sows. An important fact in the etiology of disease is that it only appears if the presence of the causal agent coexists with other secondary microorganisms.

The mechanism of action of *Serpulina* is little known but two powerful toxins with cytotoxic activity have been described that lead to the presentation of failure in the mechanism of epithelial sodium and chlorine transport, and to colonic malabsorption processes, thus resulting in the diarrhea typical of the process. Pregnant sows constitute an important reservoir of microbes from which sucklings are infected through feces. The first stage of the growth period is the period most often affected. From this period on, fecal contamination between animals continues. The incubation period is about 10-14 days, although it can reach 3 weeks. The clinical symptoms appear cyclically, reappearing at intervals of 3-4 weeks and reinfecting animals due to the persistence of *Serpulina* in the environment and in clinically asymptomatic carrier animals. Stressors of nutritional, environmental, or sanitary type activate and aggravate the disease.

Clinically, at onset a typical dark diarrhea of greyish yellow colour appears, with body temperatures of 40 to 40.5 °C, followed by more diarrhea containing large amounts of mucus and blood. Consequently, dehydration, weight loss, uncoordinated movements, acidosis, hypercalcemia, and death appear. The typical lesions are located in the large bowel. It is characterized by hyperemia and edema of the intestinal mucosa, inflammation of the submucosal glands, fibrinous exudates, and superficial necrosis. The expansion of the disease is considerable and it has been quantified as the most frequent bacterial process in young pigs after colibacillosis. The economic consequences of swine dysentery are extraordinarily important due to the mortality, morbidity, poor growth rate, and impaired conversion rates observed with it.

For the treatment of the disease, various authors recommend the administration of Macrolides (lincomycin, spectinomycin, tiamulin, tylosin, spiramycin), Tetracycline, Neomycin, Gentamicin, Bacitracin and Sulfonamides. The reduction of therapeutic options for the prevention and cure of the process due to prohibition of the use of organic arsenicals, carbadox, olaquindox, dimetridazole, nitrofurans, virginiamyein, etc. allows the evaluation, thanks to its confirmed efficacy, of sulfadimidine in the prevention and cure of swine dysentery.

The inventive administration of non-absorbable SMAA is very advantageous considering the need for medicated feed that can be administered until advanced phases of growth without running the risk of exceeding the maximum residue limit. Practical conclusions regarding the prevention and treatment of the disease include the decisive role of secondary agents or complications in triggering the pathogenic effect of the causal agent and location of the lesion in the large bowel. As a result, treatment with a macrolide and therapeutic agents that prevent the development of secondary agents is suitable. Among them, sulfonamides seem particularly useful because they exercises their activity exclusively in the large bowel, where the characteristic lesions are located since it is not absorbed in the small intestine.

When using SMAA, the following advantages are observed:
- Possibility to administer medicated feed up to slaughter because the sulfadimidine amide does not leave residues in carcasses.
- Its broad spectrum of activity includes Gram-positive and Gram-negative bacteria and *Eimeria* protozoa, which are responsible for infectious digestive processes during the growth phase.
- Maximal activity throughout the gastrointestinal tract.
- No risk of renal intoxication, even when used at high doses, due to the absence of intestinal absorption.
- Possibility of reducing doses and saving medication costs.
- Because of its activity spectrum, SMAA is an efficacious alternative to furans, which were widely used in feed for swine which until they were prohibited.

Although establishing a dose for the administration of a therapeutic molecule is always difficult, because therapeutic efficacy not depend only on its efficacy at a given dose, but also on the state of the animals, management, sanitary conditions of operation, feeding etc., good results were obtained with SMAA in swine at doses of 550-600 g of SMAA per ton of feed. The association of SMAA with macrolides is interesting, but it is extremely important to consider the withdrawal period of the macrolide to avoid the accumulation of residues in carcasses.

### Bovine livestock:

Infectious pathology in suckling calves during the first 10-12 days at the farm generally appears as a complex digestive problem. Evidently, its presentation is favored by an insufficient provision of calostrum, the stress of transport, the administration of milk products that are poorly digestible by the calf enzyme system (milk without casein and with nondairy proteins), poor management of milk supplies (concentration, temperature etc.), unfavorable environmental conditions etc. However, the existence of an infectious agent is clear, whether it comes from the mother, other bull calves, the environment or the process of denaturalizing the milk and sera used in the preparation of milk substitutes. This bacterial flora is represented mainly by different serotypes of *Escherichia coli,* without overlooking *Clostridium, Salmonella* and viruses.

In view of these circumstances, since it is not possible in practice to avoid coadjuvant factors or infectious agents, it is clear that protective measures must be taken because of the diverse microorganisms that constitute the etiology of the diarrheic syndrome. Given the large variety of pathogenic flora that can affect the process and its location in the intestine, a medication should have a broad spectrum of activity. Another factor to consider is the widespread and frequent bacterial resistance to antibiotics, which makes the use of alternative medicaments increasingly necessary. Among them, sulfadimidine is emphasized as active against Gram-negative and Gram-positive microorganisms and practically nontoxic.

In order to take maximum advantage of this sulfonamide in the diarrhea syndrome of suckling bull calves and minimize risks, the use of soluble SMAA is especially advisable, when the route of administration is a milk substitute, and SMAA is used when the medication is administered in more advanced phases of growth in starter feed. By administering the medication through milk substitutes, its action on the intestine and high concentration in feces is assured, making its activity "in situ" very effective. In addition to its total stability in milk substitutes, non-absorbability, non-toxicity, activity at low concentrations and low cost in terms of a liter of prepared artificial milk, its perfect dispersion should be noted, which ensures its activity in every section of the digestive tract and throughout the intestinal mucosa, precisely where the pathogenic microbes (*E*. *coli*) that cause the diarrhea are located. With a milk substitute, a dosage from 5 to 8 kg of soluble SMAA per ton of milk substitute is preferred. With drinking water, a dosage of 500 to 1100 g of soluble SMAA per 2000 liters of drinking water is preferred. With food, from 500 to 1000 g of SMAA per ton of feed is preferred.

### Synthesis of compounds of the invention:

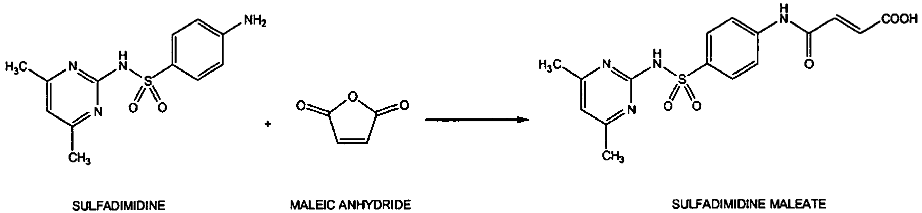

EXAMPLE 1: *4,6-dimethyl-2-N⁴-maleylsulfanilamidopyrimidine.-* 3.52 grams of maleic anhydride was added to a boiling suspension of 10 grams of 4,6-dimethyl-2-sulfanilamidopyrimidine in 100 cc. of alcohol, The mixture was then refluxed for five minutes after the addition was complete at which time all of the solids were in solution. The solution was then cooled and diluted with an equal volume of water. The white solid precipitate which formed was filtered and recrystallized from dilute alcohol yielding 4,6-dimethyl-2-N⁴-maleylsulfanilamidopyrimidine, melting at 203-206° C.

EXAMPLE 2. *4,6-dimethyl-2-N⁴-phthalylsulfanilamidopyrimidine.-5.32* grams of phthalic anhydride was added to a boiling suspension of 10 grams of 4,6-dimethyl-2-sulfanilamidopyrimidine in 100 cc. of acetone. The reaction product was worked up as in Example 1, yielding 2-N⁴-phthalylsulphanilamidothiazole, melting at 188-190° C.

EXAMPLE 3. *4,6-dimethyl-2-N⁴succinylsulfanilamidopyrimidine.*-By replacing the maleic anhydride in Example 1 by the equivalent amount of succinic anhydride, there is obtained 2,4-dimethyl-2-N⁴-succinylsulfanilamidopyrimidine melting at 226-228° C.

Example 4: The carboxylate form of any of the compounds of the invention may be prepared by suspending the compound in water and then adding the equivalent amount of, for example, anhydrous sodium carbonate. The solution is preferably filtered and from the filtrate the highly soluble sodium salt can be isolated, for example, by adding an equal volume of alcohol and pouring the resulting solution into about 10 volumes of acetone. The substance thrown out of solution is permitted to settle and the supernatant liquid withdrawn and the residue preferably treated several times with fresh acetone. After decanting the acetone from the last treatment, the resulting desired sodium salt may be dried preferably under vacuum.

### Figures:

Figure 1 shows the activity of several coccidiostatics during the evolutive cycle of *Eimeria.* Darker areas correspond to phases with maximum therapeutic activity.

## Claims

1. A dicarboxylic acid amide of sulfadimidine.

2. The compound of claim 1, wherein the compound is essentially non-absorbable in the intestine.

3. The compound of claim 1, wherein the dicarboxylic acid is selected from the group consisting of oxalic, malonic, succinic, glutaric, adipic, fumaric, maleic, malic, aspartic, citromaleic, pyrotartaric, ethylsuccinic, ketosuccinic, citraconic, mesaconic and tartaric acid and their substituted derivatives.

4. The compound of any of claims 1 to 3, wherein the compound is a watersoluble salt.

5. The compound of any of claims 1 to 4, which is an amide of a derivative of sulfadimidine.

6. A medical preparation for the treatment of diseases of the digestive tract of animals comprising a compound of any of claims 1 to 5.

7. The medical preparation of claim 6 comprising at least one other antibiotic and/or chemotherapeutic agent.

8. The medical preparation of claim 7, wherein the other antibiotic or chemotherapeutic agent is selected from the group consisting of chlortetracycline, oxytetracycline, colistin, tetracycline, tylosin, gentamicin or neomycin.

9. The medical preparations of any of claims 6 to 8 in the form of tablets, capsules, powders or solutions.

10. A food preparation for animals comprising a compound of any of claims 1 to 5.

11. The food preparation of claim 10, wherein the compound is comprised at amounts of 0.1 to 2, preferably 0.5-1 g/kg of feed.

12. A beverage preparation for animals comprising a compound of any of claims 1 to 5.

13. The beverage preparation of claim 12, wherein the compound is comprised at concentrations of 0.05 to 1, preferably 0.25 to 0.55 g/l.

14. The use of a compound of any of claims 1 to 5 for the preparation of a medicament against diseases of the digestive tract in animals.

15. The use of claim 14, wherein the absorption of the compound in the intestine is less than 0.005%, preferably less than 0.001%.

16. The use of claim 14 or 15, wherein the diseases are caused by or associated with the infection of the digestive tract by parasites and/or bacteria.

17. The use of any of claims 14 to 16, wherein the diseases are intestinal coccidiosis, digestive syndrome, enterotoxemia (overeating disease), dysentery, enteric colibacillosis (watery diarrhea), an infection by *Salmonella, E. coli, Streptococcus, Staphylococcus* and/or *Proteus* and/or diseases caused by infection with *Coccidia, Eimeria* and/or *Clostidium perfingens.*

18. The use of any of claims 14 to 17, wherein the animals are pigs, rabbits, poultry, especially chicken, turkey and/or ruminants, especially cattle and sheep.

19. A method for obtaining a medical preparation of claim 6 or a beverage preperation of claim 12 comprising the step of dissolution of the compound in water or a milk substitute.

20. A method for synthesis of a compound of any of claims 1 to 5, comprising the steps of
- adding an anhydride of a dicarboxylic acid to a heated solution of sulfadimidine or a derivative of sulfadimidine and a solvent,
- stirring and cooling,
- precipitating the compound by addition of water
- optionally filtering and recrystallizing the compound.

## Patentansprüche

1. Dicarbonsäureamid von Sulfadimidin.

2. Verbindung gemäß Anspruch 1, wobei die Verbindung im Darm im Wesentlichen nicht resorbierbar ist.

3. Verbindung gemäß Anspruch 1, wobei die Dicarbonsäure aus der Gruppe ausgewählt ist, die aus Oxal-, Malon-, Bernstein-, Glutar-, Adipin-, Fumar-, Malein-, Äpfel-, Asparagin-, Citromalein-, Brenzwein-, Ethylbernstein-, Oxalessig-, Citracon-, Mesacon- und Weinsäure und ihren substituierten Derivaten besteht.

4. Verbindung gemäß einem der Ansprüche 1 bis 3, wobei die Verbindung ein wasserlösliches Salz ist.

5. Verbindung gemäß einem der Ansprüche 1 bis 4, bei der es sich um ein Amid eines Derivats von Sulfadimidin handelt.

6. Medizinisches Präparat für die Behandlung von Erkrankungen des Verdauungstrakts von Tieren, umfassend eine Verbindung gemäß einem der Ansprüche 1 bis 5.

7. Medizinisches Präparat gemäß Anspruch 6, das wenigstens ein weiteres Antibiotikum und/oder Chemotherapeutikum umfasst.

8. Medizinisches Präparat gemäß Anspruch 7, das das weitere Antibiotikum oder Chemotherapeutikum aus der Gruppe ausgewählt ist, die aus Chlortetracyclin, Oxytetracyclin, Colistin, Tetracyclin, Tylosin, Gentamicin oder Neomycin besteht.

9. Medizinische Präparate gemäß einem der Ansprüche 6 bis 8 in Form von Tabletten, Kapseln, Pulvern oder Lösungen.

10. Futterpräparat für Tiere, das eine Verbindung gemäß einem der Ansprüche 1 bis 5 umfasst.

11. Futterpräparat gemäß Anspruch 10, wobei die Verbindung in Mengen von 0,1 bis 2 g, vorzugsweise 0,5 bis 1 g, pro kg Futter enthalten ist.

12. Trinkpräparat für Tiere, das eine Verbindung gemäß einem der Ansprüche 1 bis 5 umfasst.

13. Trinkpräparat gemäß Anspruch 12, wobei die Verbindung in Konzentrationen von 0,05 bis 1 g/l, vorzugsweise 0,25 bis 0,55 g/l, enthalten ist.

14. Verwendung einer Verbindung gemäß einem der Ansprüche 1 bis 5 zur Herstellung eines Medikaments gegen Erkrankungen des Verdauungstrakts bei Tieren.

15. Verwendung gemäß Anspruch 14, wobei die Resorption der Verbindung im Darm kleiner als 0,055%, vorzugsweise kleiner als 0,001%, ist.

16. Verwendung gemäß Anspruch 14 oder 15, wobei die Erkrankungen durch die Infektion des Verdauungstrakts durch Parasiten und/oder Bakterien verursacht oder damit assoziiert sind.

17. Verwendung gemäß einem der Ansprüche 14 bis 16, wobei es sich bei den Erkrankungen um Darmkokzidiose, Verdauungsstörungen, Enterotoxämie (durch Überfressen), Dysenterie, Kolibazillose (wässrigen Durchfall), eine Infektion durch *Salmonella, E. coli, Streptococcus, Staphylococcus* und/oder *Proteus* und/oder Erkrankungen, die durch Infektion mit *Coccidia, Eimeria* und/oder *Clostridium perfringens* verursacht sind, handelt.

18. Verwendung gemäß einem der Ansprüche 14 bis 17, wobei es sich bei den Tieren um Schweine, Kaninchen, Geflügel, insbesondere Hühner, Truthahn, und/oder Wiederkäuer, insbesondere Rinder und Schafe, handelt.

19. Verfahren zur Gewinnung eines medizinischen Präparats gemäß Anspruch 6 oder eines Trinkpräparats gemäß Anspruch 12, umfassend den Schritt des Auflösens der Verbindung in Wasser oder einem Milchersatz.

20. Verfahren zur Synthese einer Verbindung gemäß einem der Ansprüche 1 bis 5, umfassend die Schritte des
- Hinzufügens eines Anhydrids einer Dicarbonsäure zu einer erhitzten Lösung eines Sulfadimidins oder eines Derivats von Sulfadimidin und eines Lösungsmittels;
- Rührens und Kühlens;
- Ausfällens der Verbindung durch Zugabe von Wasser;
- gegebenenfalls Filtrierens und Umkristallisierens der Verbindung.

## Revendications

1. Amide d'acide dicarboxylique de sulfadimidine.

2. Composé selon la revendication 1, dans lequel le composé est essentiellement non absorbable dans l'intestin.

3. Composé selon la revendication 1, dans lequel l'acide dicarboxylique est choisi dans le groupe constitué des acides oxalique, malonique, succinique, glutarique, adipique, fumarique, maléique, malique, aspartique, citromaléique, pyrotartrique, éthylsuccinique, cétosuccinique, citraconique, mésaconique et tartrique et de leurs dérivés substitués.

4. Composé selon l'une quelconque des revendications 1 à 3, dans lequel le composé est un sel soluble dans l'eau.

5. Composé selon l'une quelconque des revendications 1 à 4, qui est un amide d'un dérivé de sulfadimidine.

6. Préparation médicale pour le traitement de maladies du tube digestif d'animaux comprenant un composé de l'une quelconque des revendications 1 à 5.

7. Préparation médicale selon la revendication 6, comprenant au moins un autre agent antibiotique et/ou chimiothérapeutique.

8. Préparation médicale selon la revendication 7, dans laquelle l'autre agent antibiotique ou chimiothérapeutique est choisi dans le groupe constitué de la chlortétracycline, de l'oxytétracycline, de la colistine, de la tétracycline, de la tylosine, de la gentamicine ou de la néomycine.

9. Préparations médicales selon l'une quelconque des revendications 6 à 8, sous la forme de comprimés, capsules, poudres ou solutions.

10. Préparation alimentaire pour animaux comprenant un composé de l'une quelconque des revendications 1 à 5.

11. Préparation alimentaire selon la revendication 10, dans laquelle le composé est compris en des quantités de 0,1 à 2, de préférence de 0,5 à 1 g/kg de nourriture.

12. Préparation de boisson pour animaux comprenant un composé de l'une quelconque des revendications 1 à 5.

13. Préparation de boisson selon la revendication 12, dans laquelle le composé est compris en des concentrations de 0,05 à 1, de préférence de 0,25 à 0,55 g/L.

14. Utilisation d'un composé de l'une quelconque des revendications 1 à 5, pour la préparation d'un médicament contre des maladies du tube digestif chez les animaux.

15. Utilisation selon la revendication 14, dans laquelle l'absorption du composé dans l'intestin est inférieure à 0,005 %, de préférence inférieure à 0,001 %.

16. Utilisation selon la revendication 14 ou 15, dans laquelle les maladies sont provoquées par ou associées à l'infection du tube digestif par des parasites et/ou des bactéries.

17. Utilisation selon l'une quelconque des revendications 14 à 16, dans laquelle les maladies sont la coccidiose intestinale, un syndrome digestif, l'entérotoxémie (maladie de suralimentation), la dysenterie, la colibacillose entérique (diarrhée aqueuse), une infection à *Salmonella, E. coli*, *Streptococcus, Staphylococcus* et/ou *Proteus* et/ou des maladies provoquées par une infection à *Coccidia, Eimeria* et/ou *Clostridium perfringens.*

18. Utilisation selon l'une quelconque des revendications 14 à 17, dans laquelle les animaux sont des cochons, des lapins, de la volaille, en particulier les poulets, les dindes et/ou des ruminants, en particulier les bovins et les ovins.

19. Procédé d'obtention d'une préparation médicale de la revendication 6 ou d'une préparation de boisson de la revendication 12, comprenant l'étape de dissolution du composé dans l'eau ou un succédané du lait.

20. Procédé de synthèse d'un composé de l'une quelconque des revendications 1 à 5, comprenant les étapes
- d'ajout d'un anhydride d'un acide dicarboxylique à une solution chauffée de sulfadimidine ou d'un dérivé de sulfadimidine et d'un solvant,
- d'agitation et de refroidissement,
- de précipitation du composé par l'ajout d'eau,
- de filtrage et de recristallisation facultatifs du composé.
